# EUROPÄISCHE PATENTSCHRIFT

(11) **EP 2 797 423 B1**
(45) Veröffentlichungstag und Bekanntmachung des Hinweises auf die Patenterteilung: **20.04.2016**
(21) Anmeldenummer: 12791427.3
(22) Anmeldetag: 15.11.2012
(51) Int. Cl.: A23D 7/00, A23D 7/02, A61K 8/06

(54) **VEGANE EMULSION**
VEGAN EMULSION
EMULSION VÉGÉTALIENNE

(30) Priorität: 15.11.2011 DE 102011118500
(43) Veröffentlichungstag der Anmeldung: 05.11.2014
(73) Patentinhaber: Starck's Food GmbH & Co. KG, 22089 Hamburg (DE)
(72) Erfinder: STARCK, Jürgen, 21509 Glinde (DE)
(74) Vertreter: Sasse, Stefan
(86) Internationale Anmeldenummer: PCT/EP2012/004740
(87) Internationale Veröffentlichungsnummer: WO 2013/072056

(56) Entgegenhaltungen:
- EP-A1- 0 197 776
- EP-A1- 0 892 035
- WO-A1-85/03646
- WO-A1-2006/012723
- US-A- 3 143 475
- US-A- 5 322 704
- US-A- 5 942 216

## Beschreibung

Die vorliegende Erfindung betrifft eine vegane Emulsion, insbesondere eine Lebensmittelemulsion und/oder eine kosmetische Emulsion, sowie ein Herstellungsverfahren dieser Emulsion. Bei der erfindungsgemäßen Emulsion handelt es sich um eine multiple Emulsion, bei der mindestens 90% der Emulsionströpfchen der multiplen Emulsion einen Durchmesser von 2-10 µm, vorzugsweise 4-8 µm, aufweisen.

Aus dem Stand der Technik sind vergleichbare Emulsionen, insbesondere aus dem Bereich der Lebensmittel oder Kosmetika bekannt. Bekannte Lebensmittelemulsionen sind beispielsweise Milch, Mayonnaisen, Salatdressings oder aber auch Margarinen oder Speiseeis. Im Bereich der Kosmetik sind insbesondere Cremes und Lotions bekannt.

Viele der bekannten Emulsionen können jedoch nicht als vegan bezeichnet werden, da diese häufig Bestandteile, wie Milch- oder Molkeproteine oder Eigelb oder von Eigelb abgeleitete Emulgatoren, wie Ei-Lecithin, aufweisen. Für eine vegane Lebensweise - einen vollständigen Verzicht auf tierische Produkte - sind solche Emulsionen weitestgehend ungeeignet. Häufig weisen derartige Emulsionen daher Sojalecithin als Emulgator auf, welches jedoch aufgrund der Herkunft - gewonnen aus der Sojabohne - nachteilig sein kann, da Sojalecithin häufig weitere Sojaproteine aufweist, welche als Allergene wirken und Allergien auslösen können.

Vegane Emulsionen, beispielsweise frisch angerührte Salatdressings, bestehend aus einer wässrigen Phase und Öl, sind häufig nicht besonders stabil, dass heißt, es kommt zu einem Emulsionsbruch und/oder einer Phasentrennung. Das heißt in anderen Worten die vorher fein verteilten Phasen der Emulsion trennen sich wieder in wässrige Phase und Öl-Phase.

Der vorliegenden Erfindung liegt daher die Aufgabe zugrunde, eine besonders langzeit- und lagerstabile vegane Emulsion herzustellen, die insbesondere für den Verkauf im Einzelhandel nicht gekühlt werden muss. Die Langzeitstabilität einer Emulsion, gemeint ist hier eine Lagerungszeit von mindestens einigen Monaten, vorzugsweise mindestens zwei bis zwölf Monaten, ist insbesondere bei ungekühlten Nahrungsmittelemulsionen aber auch bei Emulsion in der Kosmetik von besonderer Relevanz. Eine weitere erfindungsgemäße Aufgabe kann die Bereitstellung einer veganen, cholesterinarmen, und allergenarmen Lebensmittelemulsion sein.

Eine weitere Aufgabe der Erfindung ist es, ein Herstellungsverfahren für eine langzeit- und lagerstabile und vegane Emulsion bereitzustellen. Eine weitere Aufgabe kann es sein eine besonderes homogene Emulsion herzustellen, die insbesondere auch nach einem Pasteurisierungsschritt im Wesentlichen ihre Stabilität und/oder ihre Struktur beibehält. So kann es ebenfalls eine erfindungsgemäße Aufgabe sein, die mikrobiologische Qualität einer veganen Emulsion zu verbessern.

Die erfindungsgemäßen Aufgaben werden von einer veganen Emulsion mit den Merkmalen des Anspruchs 1 oder einem Herstellungsverfahren mit den Merkmalen des Anspruchs 9 gelöst. Bevorzugte Ausgestaltungen der Erfindung sind in den Unteransprüchen angegeben.

Erfindungsgemäß ist eine vegane Emulsion bestehend aus mindestens einem pflanzlichen Öl, Wasser und mindestens einem pflanzlichen Hilfsstoff, dadurch gekennzeichnet, dass die Emulsion eine multiple Emulsion ist, wobei mindestens 90% (Anzahlverteilung) der Emulsionströpfchen der multiplen Emulsion einen Durchmesser von 2-10 µm, vorzugsweise 4-8 µm, aufweisen.

Mit der Bezeichnung vegan werden im Allgemeinen Produkte bezeichnet, die keine Bestandteile aus Tieren aufweisen. Gemeint ist damit, dass insbesondere keine Produktbestandteile eine tierische Herkunft besitzen. Insbesondere enthalten vegane Emulsionen daher keine Bestandteile die aus Tieren oder aus tierischen Produkten, wie Milch, Molke oder Ei gewonnen werden. Als Emulsion wird ein fein verteiltes Gemisch aus zwei normalerweise nicht mischbaren Flüssigkeiten, insbesondere Wasser und Öl, bezeichnet. Bekannte Emulsionen sind beispielsweise Milch, Mayonnaisen, Salatdressings oder aber auch Margarinen oder Speiseeis. Die erfindungsgemäße vegane Emulsion kann als Ausgangstoff einer veganen Lebensmittelemulsion, beispielsweise eines Salatdressings, eines "Mayonnaise-ähnlichen" Produkts, einer Sauce, eines veganen Speiseeis oder eines Brotaufstrichs oder ähnlicher Produkte, verwendet werden. In einer anderen Ausführungsform kann die vegane Emulsion als Ausgangstoff einer Kosmetik, beispielsweise einer Creme, einer Lotion oder eines Duschgels oder ähnlicher Produkte, verwendet werden.

Die erfindungsgemäße Emulsion besteht aus mindestens einem pflanzlichen Öl, beispielsweise Sonnenblumen-, Oliven-, Raps-, Lein- oder Palmöl. Es können aber auch andere pflanzliche Öle verwendet werden. Vorzugsweise werden möglichst allergen- und/oder cholesterinarme pflanzliche Öle verwendet. Allergenarm in diesem Zusammenhang heißt, dass für insbesondere weit verbreitete Nahrungsmittel-Allergien im Wesentlichen keine diese Allergien auslösende Stoffe, insbesondere Proteine, enthalten sind. Weit verbreitete Nahrungsmittel-Allergien sind beispielsweise Nussallergien (Erdnüsse, Walnüsse, Sesam, Mandeln), Eigelb- und Eiweiß-Allergien oder Milchallergien, wie beispielsweise eine Lactoseintoleranz., sowie Allergien gegenüber Getreidesorten oder Sojabohnen. Vorzugsweise werden also pflanzliche Öle und pflanzliche Hilfsstoffe für die Herstellung einer erfindungsgemäßen Emulsion verwendet, die keine der obengenannten Nahrungsmittel-Allergie auslöst. In einer besonders bevorzugten Ausgestaltung weist die Emulsion daher keine Sojaproteine und/oder keinen aus Soja abgeleiteten oder gewonnenen Emulgator, insbesondere kein Sojalecithin, auf. Vegane Emulsionen, insbesondere Mayonnaise-ähnliche Produkte, haben gegenüber Emulsionen, wie Mayonnaisen, mit Eigelb als Emulgator häufig einen geschmacklichen Nachteil, da die bekannten veganen Emulsionen in der Regel Sojabasierende Emulgatoren verwenden, die häufig auch einen von der Soja stammenden Bitterstoff aufweisen, der so in das Endprodukt gelangt. Insbesondere durch den Verzicht auf Soja als Zutat kann so auch ein geschmacklicher Vorteil gegenüber den bisherigen veganen Emulsionen auf Soja-Basis erzielt werden.

Der erfindungsgemäße mindestens eine pflanzliche Hilfsstoff kann insbesondere aus der Gruppe der pflanzliche Emulgatoren, der pflanzlichen Verdickungsmittel oder der pflanzlichen Geschmackstoffe, beispielsweise aus der Gruppe der Gewürze, ausgewählt werden. Vorzugsweise kann als pflanzlicher Emulgator Senfmehl verwendet werden. Dieses Senfmehl kann vorzugsweise sehr fein gemahlen sein, dass heißt, vorzugsweise sind die Senfmehlpartikel in einer Siebanalyse nicht größer als 2 bis 20 µm, besonders vorzugweise nicht größer als 2 bis 10 µm oder 4 bis 8 µm. Pflanzliche Verdickungsmittel können Johannisbrotkernmehl, Carragen, Guarkernmehl, Alginate, mikrokristalline Cellulose, Methylcellulose oder andere Celluosederivate sein. Alternativ können aber auch Verdickungsmittel wie Xanthan, Gellan oder Agar oder andere bakteriell hergestellte oder aus Bakterien gewonnene Verdickungsmittel verwendet werden. Die Partikelgröße der pflanzlichen Hilfsstoffe kann beispielsweise mit Sieben mit einer entsprechenden Maschenweite bestimmt werden.

Die erfindungsgemäße Emulsion ist eine multiple Emulsion. Eine multiple Emulsion kann beispielsweise eine Wasser-Öl-Wasser Emulsion oder eine Öl-Wasser-Öl Emulsion sein. Alle multiplen Emulsionen bestehen mindestens aus einer inneren, ersten Emulsion und einer äußeren, zweiten Emulsion. Vorzugsweise ist die erfindungsgemäße Emulsion eine Wasser-Öl-Wasser Emulsion. Durch die Verwendung einer Wasser-Öl-Wasser Emulsion können verschiedene Vorteile erzielt werden. So kann beispielsweise der Anteil des pflanzlichen Öls an der Emulsion reduziert werden. Die Reduzierung des Öl-Anteils kann nicht nur bedeuten, dass die Emulsion Fett- oder Öl-reduziert ist und die Emulsion somit kalorienärmer ist, sondern gleichzeitig kann, im Vergleich zum Wasser teueres, Öl eingespart werden und somit eine Reduzierung der Materialkosten erzielt werden.

Des Weiteren kann bei der Verwendung einer multiplen Emulsion besonders vorteilhafte Anwendungen und Wirkungen ermöglicht werden. So kann es beispielsweise vorteilhaft sein, bei einer Wasser-Öl-Wasser Emulsion in die innere, wässrige Phase der ersten Emulsion Geschmackstoffe, Farbstoffe oder biologisch aktive Stoffe zu lösen. Eine solche innere Phase ist in der Regel bei multiplen Emulsionen besonders geschützt, da die wässrige Phase innerhalb eines Emulsionströpchen der multiplen Emulsion, vorliegt. Vorzugsweise können so bei einer Wasser-Ol-Wasser Emulsion, beispielsweise im kosmetischen Bereich, in der inneren wässrigen Phase aktive Substanzen oder Wirkstoffen vorliegen, die so beispielsweise vor Oxidation geschützt werden.

Erfindungsgemäß weisen mindestens 90%, gemeint ist eine Anzahlverteilung, der Emulsionströpfchen der multiplen Emulsion einen Durchmesser von 2-10 µm, vorzugsweise 4-8 µm, auf. Vorzugsweise entspricht die EmulsionstropfendurchmesserVerteilung im Wesentlichen einer gaußschen Normalverteilung. Dass heißt die meisten Emulsionströpchen haben einen Durchmesser von 5-7 µm. Bei der Betrachtung einer solchen Normalverteilung wäre beispielsweise ein Scheitelpunkt der Glockenkurve bei ungefähr 5-7 µm Durchmesser zu beobachten.

Besonders vorzugsweise haben mindestens 90% der Emulsionströpchen einen Durchmesser von 3-7 µm, so dass die meisten Emulsionströpchen ungefähr 5 µm groß sind.
Vorzugsweise kann die vegane Emulsion, insbesondere die zweite Emulsion der multiplen Emulsion mittels einer metallischen Lochfolie oder mittels einer Gewebestruktur hergestellt werden. Denkbar wäre es aber auch die mindestens zweite Emulsion der multiplen Emulsion mittels einer keramischen Membran, beispielsweise einer Cross-flow Membran herzustellen. Vorzugsweise wird die zweite Emulsion mittels einer Lochfolie, die Löcher mit einem Durchmesser von 2-10 µm aufweist, oder eines Gewebes, das eine Maschenweite von 2 bis 10 µm aufweist. Vorzugsweise ist die Lochfolie, das Gewebe oder die keramische Membran derart ausgebildet und/oder an das Herstellungsverfahren angepasst, dass ein im Wesentlichen kontinuierliches Herstellungsverfahren und/oder eine Sterilisation oder Reinigung möglich ist. Vorzugsweise ist die Lochfolie und/oder das Gewebe aus einen metallischen Material, das sich Dampfsterilisieren lässt. Besonders vorzugsweise ist die Lochfolie, das Gewebe oder die keramische Membran derart ausgebildet, dass die durchfließenden Flüssigkeiten, insbesondere die erste Emulsion, möglichst wenig haftet oder eine möglichst geringe Reibung erzeugt wird, beispielsweise dadurch, dass sie eine Nanostruktur, insbesondere ähnlich eines Lotusblattes, aufweisen.

Ebenfalls erfindungsgemäß ist ein Verfahren zur Herstellung einer veganen Emulsion, insbesondere nach Anspruch 1, wobei das Verfahren mindestens folgende Schritte aufweist: a) Herstellen einer erste Emulsion aus Öl und Wasser, b) Dispergieren der pflanzlichen Hilfsstoffe in einer wässrigen Phase oder in der ersten Emulsion, c) Herstellen einer multiplen Emulsion aus der ersten Emulsion und der wässrigen Phase, dass dadurch gekennzeichnet, dass mindestens 90% der Emulsionströpfchen der multiplen Emulsion einen Durchmesser von 2-10 µm, vorzugsweise 4-8 µm, aufweisen. In einem ersten Schritt des erfindungsgemäßen Herstellungsverfahrens wird eine erste Emulsion aus, insbesondere einem pflanzlichen, Öl und Wasser hergestellt. Die erste Emulsion kann insbesondere mittels Ultraschall oder mittels eines Homogenisators hergestellt wird. Mittels Ultraschall kann bedeuten, dass ein Ultraschallgeber Schallwellen in die wässrige Phase und/oder das Öl abgibt und die Schallwellen eine im Wesentlichen gleichmäßige feine Verteilung der Flüssigkeiten bewirkt, eine Emulsion entsteht. Ein Homogenisator kann insbesondere eine Mischvorrichtung, beispielsweise ein Ultrathurax-Homogenisator, sein, die die beiden Phasen mittels eines mechanischen Mischvorgangs vermischt, so dass eine Emulsion entsteht.
In einem weiteren Schritt wird mindestens ein pflanzlicher Hilfsstoffe der in die wässrige Phase oder in die erste Emulsion dispergiert. Pflanzliche Hilfsstoffe sind insbesondere solche, die für die erfindungsgemäße vegane Emulsion verwendet werden. Vorzugsweise wird bereits bei dem Schritt a) Herstellungsschritt der ersten Emulsion ein Geschmacksträger, ein Aromastoff, ein Farbstoff oder eine biologisch aktive Substanz zugesetzt wird. Biologisch aktive Substanzen können in diesem Zusammenhang Wirkstoffe, insbesondere der Kosmetik, und/oder Substanzen sein, die Kosmetik eine Wirkung, beispielsweise auf die Haut, haben sollen. Denkbar sind aber auch Vitamine, Proteine, wie Enzyme, Co-Faktoren, Aminosäuren oder cholesterinsenkende Wirkstoffe. In einem nächsten Schritt c) passiert die erste Emulsion eine, insbesondere metallische, Lochfolie, eine Gewebestruktur, beispielsweise ein Sieb, oder eine keramische Membran. Insbesondere während des Austritts in die wässrige Phase wird dann die multiple Emulsion gebildet. Dem Verfahrensschritt c) kann sich ein weiterer Schritt d) anschließen. In diesem Schritt kann die multiple Emulsion thermisch behandelt, beispielsweise pasteurisiert, werden.

Das Herstellungsverfahren kann um weitere Schritte ergänzt werden. Insbesondere kann die erfindungsgemäße Emulsion, vorzugsweise eine Lebensmittelemulsion, während einer weiteren Verarbeitung zu einem Endprodukt mit weiteren Zutaten, insbesondere Geschmacksstoffen, wie Salz, Zucker oder Gewürzen, oder organischen Säuren, wie Essigsäure, Zitronensäure oder Ascorbinsäure vermischt werden.

Diese und weitere Merkmale und Vorteile der Erfindung werden mit Bezug auf die beigefügte Zeichnung eines Ausführungsbeispiel eines erfindungsgemäßen Herstellungsschritte weiter beschrieben. In den Zeichnungen zeigen
- **Figur 1,**: einen Emulsionstropfen eine multiplen Emulsion
- **Figur 2,**: einen Querschnitt einer Lochfolie, an dem sich ein Emulsionstropfen der zweiten Emulsion der multiplen Emulsion bildet.

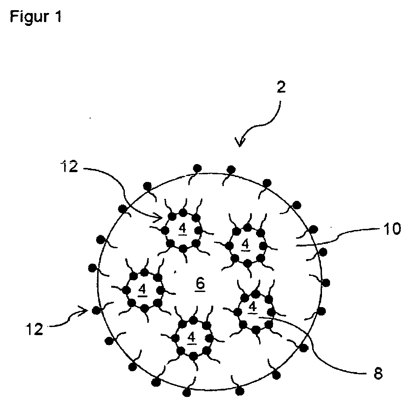

Die **Figur 1** zeigt einen Emulsionstropfen **2** einer multiplen Emulsion. Erfindungsgemäß haben mindestens 90% der Emulsionströpfchen einen Durchmesser von 2-10 µm, vorzugsweise 4-8 µm. Der Emulsionstropfen **2** der multiplen Emulsion weist einen inneren Emulsionstropfen **8** einer ersten Emulsion auf, der eine innere wässrige Phase **4** und eine äußere Öl-Phase **6** aufweist. Diese erste Öl-Wasser-Emulsion wird in einem Schritt a) des Herstellungsverfahrens hergestellt. In Schritt c) wird die zweite, äußere Emulsion hergestellt. Die Emulsionstropfen **10** der zweiten Emulsion weisen eine innere Öl-Phase **6** auf, die gleichzeitig die äußere Öl-Phase **6** der ersten Emulsion ist. Die äußerer Phasen der Emulsionstropfen **10** ist eine wässrige Phase, hier nicht dargestellt, die den Emulsionstropfen **10** umschließt. Der Emulsionstropfen **10** der zweiten Emulsion ist, wie hier dargestellt, gleichzeitig ein Emulsionstropfen **2** der multiplen Emulsion. An den jeweiligen Grenzflächen zwischen einer Öl-Phase **6** und einer wässrigen Phase **4** ordnen sich bevorzugt emulgierende Hilfsstoffe **12** an.

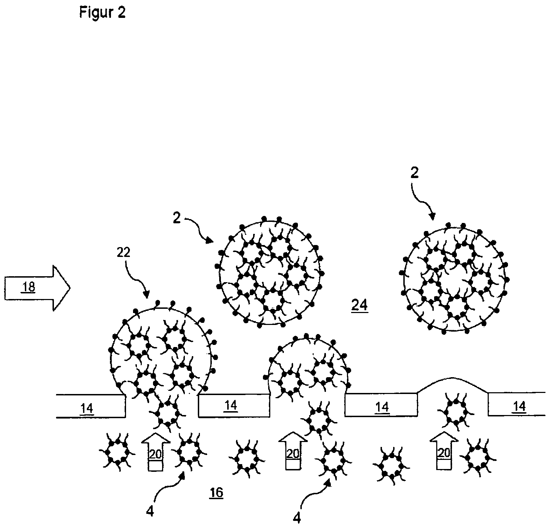

Die **Figur 2** zeigt einen Querschnitt einer Lochfolie **14** an dem sich ein Emulsionstropfen **2** der zweiten Emulsion der multiplen Emulsion **24** bildet. Die Emulsionströpfen **2** werden während und/oder nach der Passage durch die Lochfolie **14** gebildet. In einer alternativen Ausführungsform kann anstatt der Lochfolie ein Gewebe oder eine keramische Membran verwendet werden. Die Struktur, gekennzeichnet mit dem Bezugszeichen **14,** kann daher auch ein Gewebe oder eine Membran darstellen.
Die erste Emulsion **16,** eine Öl-Wasser Emulsion, fließt, wie durch die Blockpfeile **20** dargestellt, aus einer Vorlage durch die Lochfolie, das Gewebe oder die Membran **14** und wird anschließend mit einer wässrigen Phase vermischt. Die wässrige Phase fließt, wie durch den Blockpfeil **18** dargestellt, über die Lochfolie, das Gewebe oder die Membran **14.** An der Grenzfläche zwischen der ersten Emulsion **16** und der wässrigen Phase bildet sich ein Emulsionstropfen **2,** wie mit dem Bezugszeichen **22** dargestellt, so dass eine die multiple Emulsion **24,** hier dargestellt eine Wasser-Öl-Wasser Emulsion, entsteht.

## Patentansprüche

1. Vegane Emulsion bestehend aus mindestens einem pflanzlichen Öl, Wasser und mindestens einem pflanzlichen Hilfsstoff, **dadurch gekennzeichnet, dass** die Emulsion eine multiple Emulsion (24) ist, wobei mindestens 90% der Emulsionströpfchen der multiplen Emulsion (24) einen Durchmesser von 2-10 µm aufweisen.

2. Emulsion nach Anspruch 1, **dadurch gekennzeichnet, dass** mindestens 90% der Emulsionströpfchen einen Durchmesser 4-8 µm aufweisen.

3. Emulsion nach einem der vorhergehenden Ansprüche, **dadurch gekennzeichnet, dass** die multiple Emulsion (24) eine Wasser-Öl-Wasser Emulsion ist.

4. Emulsion nach einem der vorhergehenden Ansprüche, **dadurch gekennzeichnet, dass** die Emulsion eine Lebensmittelemulsion und/oder eine kosmetische Emulsion ist.

5. Emulsion nach einem der vorhergehenden Ansprüche, **dadurch gekennzeichnet, dass** die Emulsion kein Eigelb, oder keinen aus Eigelb abgeleiteten Emulgator aufweist.

6. Emulsion nach einem der vorhergehenden Ansprüche, **dadurch gekennzeichnet, dass** die Emulsion kein Sojaprotein, oder keinen aus Soja abgeleiteten Emulgator, insbesondere kein Sojalecithin, aufweist.

7. Emulsion nach einem der vorhergehenden Ansprüche, **dadurch gekennzeichnet, dass** die Emulsionstropfendurchmesserverteilung im Wesentlichen einer gaußschen Normalverteilung entspricht.

8. Emulsion nach einem der vorhergehenden Ansprüche, **dadurch gekennzeichnet, dass** die Hilfsstoffe eine Partikelgröße von 4-10 µm, vorzugsweise 4-8 µm, aufweisen.

9. Emulsion nach einem der vorhergehenden Ansprüche, **dadurch gekennzeichnet, dass** mindestens die zweite Emulsion der multiplen Emulsion mittels einer Lochfolie (14) oder mittels einer Gewebestruktur hergestellt wird.

10. Verfahren zur Herstellung einer veganen Emulsion nach Anspruch 1, wobei das Verfahren mindestens folgende Schritte aufweist:
a) Herstellen einer erste Emulsion aus Öl und Wasser,
b) Dispergieren mindestens eines pflanzlichen Hilfsstoffs in einer wässrigen Phase oder in der ersten Emulsion,
c) Herstellen einer multiplen Emulsion aus der ersten Emulsion und der wässrigen Phase
**dadurch gekennzeichnet, dass** mindestens 90% (Anzahlverteilung) der Emulsionströpfchen der multiplen Emulsion einen Durchmesser von 2-10 µm, vorzugsweise 4-8 µm, aufweisen.

11. Verfahren nach Anspruch 10, **dadurch gekennzeichnet, dass** mindestens die erste Emulsion mittels Ultraschall oder mittels eines Homogenisators hergestellt wird.

12. Verfahren nach einem der Ansprüche 10 oder 11, **dadurch gekennzeichnet, dass** dem Herstellungsschritt a) der ersten Emulsion ein Geschmacksträger und/oder ein Farbstoff und/oder eine biologisch aktive Substanz zugesetzt wird.

13. Verfahren nach einem der Ansprüche 10 bis 12, **dadurch gekennzeichnet, dass** im Schritt c) die erste Emulsion eine Lochfolie (14), eine Gewebestruktur oder eine keramische Membran (14) passiert und dass während des Austritts in die wässrige Phase die multiple Emulsion (24) gebildet wird.

14. Verfahren nach einem der Ansprüche 10 bis 12, **dadurch gekennzeichnet, dass** das Verfahren zusätzlich nach Schritt c) den Schritt aufweist:
d) Pasteurisieren der multiplen Emulsion.

## Claims

1. A vegan emulsion consisting of at least one vegetable oil, water and at least one vegetable auxiliary agent, **characterized in that** the emulsion is a multiple emulsion (24), wherein at least 90% of the emulsion droplets of the multiple emulsion (24) have a diameter of 2-10 µm.

2. The emulsion according to claim 1, **characterized in that** at least 90% of the emulsion droplets have a diameter of 4-8 µm.

3. The emulsion according to any one of the preceding claims, **characterized in that** the multiple emulsion (24) is a water/oil/water emulsion.

4. The emulsion according to any one of the preceding claims, **characterized in that** the emulsion is a food emulsion and/or a cosmetic emulsion.

5. The emulsion according to any one of the preceding claims, **characterized in that** the emulsion does not have any egg yolk, or any emulsifier derived from egg yolk.

6. The emulsion according to any one of the preceding claims, **characterized in that** the emulsion does not have any soy protein, or any emulsifier derived from soy protein, especially soy lecithin.

7. The emulsion according to any one of the preceding claims, **characterized in that** the emulsion droplet diameter distribution substantially corresponds to a Gaussian normal distribution.

8. The emulsion according to any one of the preceding claims, **characterized in that** the auxiliary agents have a particle size of 4-10 µm, and preferably 4-8 µm.

9. The emulsion according to any one of the preceding claims, **characterized in that** at least the second emulsion of the multiple emulsion is produced by means of a perforated foil (14) or by means of a fabric structure.

10. A method to produce a vegan emulsion according to claim 1, wherein the method has at least the following steps:
a) Production of a first emulsion from oil and water,
b) Dispersion of at least one vegetable auxiliary agent in an aqueous phase, or in the first emulsion,
c) Production of a multiple emulsion from the first emulsion and the aqueous phase,
**characterized in that** at least 90% (numeric distribution) of the emulsion droplets of the multiple emulsion have a diameter of 2-10 µm and preferably 4-8 µm.

11. The method according to claim 10, **characterized in that** at least the first emulsion is produced by means of ultrasound or means of a homogenizer.

12. The method according to one of claims 10 or 11, **characterized in that** a flavor carrier and/or a dye and/or a biologically active substance is added to the production step a) for the first emulsion.

13. The method according to one of claims 10 to 12, **characterized in that** in step c), the first emulsion passes through a perforated foil (14), a fabric structure or a ceramic membrane (14), and that the multiple emulsion (24) is formed while exiting into the aqueous phase.

14. A method according to one of claims 10 to 12, **characterized in that** the method, in addition to step c), comprises the step:
d) Pasteurization of the multiple emulsion.

## Revendications

1. Émulsion végétalienne se composant d'au moins une huile végétale, de l'eau et d'au moins un agent auxiliaire végétal, **caractérisée en ce que** l'émulsion est une émulsion multiple (24), dans laquelle au moins 90 % des gouttelettes de l'émulsion multiple (24) présentent un diamètre de 2-10 µm.

2. Émulsion selon la revendication 1, **caractérisée en ce que** au moins 90 % des gouttelettes d'émulsion présentent un diamètre de 4-8 µm.

3. Émulsion selon l'une quelconque des revendications précédentes, **caractérisée en ce que** l'émulsion multiple (24) est une émulsion eau-huile-eau.

4. Émulsion selon l'une quelconque des revendications précédentes, **caractérisée en ce que** l'émulsion est une émulsion alimentaire et/ou une émulsion cosmétique.

5. Émulsion selon l'une quelconque des revendications précédentes, **caractérisée en ce que** l'émulsion ne présente ni du jaune d'oeuf ni un émulsifiant dérivé du jaune d'oeuf.

6. Émulsion selon l'une quelconque des revendications précédentes, **caractérisée en ce que** l'émulsion ne présente ni une protéine de soja ni un émulsifiant dérivé du soja, en particulier pas de lécithine de soja.

7. Émulsion selon l'une quelconque des revendications précédentes, **caractérisée en ce que** la distribution des diamètres des gouttes d'émulsion correspond essentiellement à une distribution normale de Gauss.

8. Émulsion selon l'une quelconque des revendications précédentes, **caractérisée en ce que** les agents auxiliaires présentent une taille de particule de 4-10 µm, de préférence 4-8 µm.

9. Émulsion selon l'une quelconque des revendications précédentes, **caractérisée en ce que** au moins la deuxième émulsion de l'émulsion multiple est préparée au moyen d'un film perforé (14) ou au moyen d'une structure tissulaire.

10. Procédé de préparation d'une émulsion végétalienne selon la revendication 1, dans lequel le procédé comporte au moins les étapes suivantes :
a) préparation d'une première émulsion se composant d'huile et d'eau,
b) dispersion d'au moins un agent auxiliaire végétal dans une phase aqueuse ou dans la première émulsion,
c) préparation d'une émulsion multiple à partir de la première émulsion et de la phase aqueuse,
**caractérisé en ce que** au moins 90 % (distribution en nombre) des gouttelettes de l'émulsion multiple présentent un diamètre de 2-10 µm, de préférence 4-8 µm.

11. Procédé selon la revendication 10, **caractérisé en ce que** au moins la première émulsion est préparée au moyen d'ultrasons ou au moyen d'un homogénéisateur.

12. Procédé selon l'une quelconque des revendications 10 ou 11, **caractérisé en ce qu'**un vecteur de goût et/ou un colorant et/ou une substance active biologique est (sont) ajouté(s) à l'étape de préparation a) de la première émulsion.

13. Procédé selon l'une quelconque des revendications 10 à 12, **caractérisé en ce que**, à l'étape c), la première émulsion traverse un film perforé (14), une structure tissulaire ou une membrane céramique (14), et **en ce que** l'émulsion multiple (24) est formée pendant la transition à la phase aqueuse.

14. Procédé selon l'une quelconque des revendications 10 à 12, **caractérisé en ce que** le procédé comporte, en plus de l'étape c), l'étape suivante :
d) pasteurisation de l'émulsion multiple.
